(19) Europäisches Patentamt
European Patent Office
Office européen des brevets

(11) Publication number: **0 409 528 A2**

(12) # EUROPEAN PATENT APPLICATION

(21) Application number: 90307747.7

(22) Date of filing: 16.07.90

(51) Int. Cl.[5]: C07F 15/00, A61K 31/28

(30) Priority: 18.07.89 US 381610

(43) Date of publication of application: 23.01.91 Bulletin 91/04

(84) Designated Contracting States: AT BE CH DE DK ES FR GB GR IT LI LU NL SE

(71) Applicant: ENGELHARD CORPORATION
Menlo Park, CN 40
Edison New Jersey 08818(US)

(72) Inventor: Amundsen, Alan R.
12 Meadowbrook Drive
Somerville, New Jersey 08876(US)
Inventor: Stern, Eric W.
234 Oak Tree Road
Mountainside, New Jersey 07092(US)
Inventor: Hollis, Leslie Steven
33 Guilford Lane
Mercerville, New Jersey 08619(US)
Inventor: Gramiccioni, Gary A.
304 Bound Brook Avenue
Piscataway, New Jersey 08854(US)

(74) Representative: Fisher, Adrian John et al
CARPMAELS & RANSFORD 43 Bloomsbury Squareare
London WC1A 2RA(GB)

(54) **Platinum triamine antitumor agents containing substituted anilines.**

(57) Platinum triamine complexes that contain aniline or substituted aniline as a ligand are used as antitumor agents. A number of such complexes are novel *per se* .

EP 0 409 528 A2

# PLATINUM TRIAMINE ANTITUMOR AGENTS CONTAINING SUBSTITUTED ANILINES.

## Field of the Invention

The present invention relates to the use of certain platinum-triamine complexes as agents for combatting malignant tumors in animals; it also relates to certain of those complexes that are new and to a process for their production.

## Background of the Invention

While cisplatin has proven effective in the treatment of a variety of forms of cancer such as testicular and ovarian carcinoma, a continuing effort has been made in analog development programs to broaden the spectrum of activity and to improve the therapeutic properties of platinum-based antitumor agents. These goals include agents that demonstrate activity in unresponsive diseases, such as colon, breast, stomach and brain cancer, and improving on the activity seen in tumor systems that presently respond to cisplatin, such as lung, bladder, and head and neck carcinomas.

During the past fifteen years, several thousand platinum complexes have been prepared and evaluated in a variety of animal tumor screens in an attempt to devise structure-activity relationships among the platinum analogs. The classical structure-activity relationships were first summarized by Cleare and Hoeschele in 1974, and since that time these guidelines have continued to dominate the search for new analogs. See, for example, Cleare et al, Bioinorg Chem. 1973 , 2 ,187. In general, platinum(II) complexes of the form cis -$PtA_2X_2$, where A is an amine ligand and X is an anionic leaving group, represent the majority of the active analogs. See Rose et al, Cisplatin, Current Status and New Developments , Academic Press, New York, New York, 1980, p. 229. The corresponding trans -diamine complexes are devoid of activity in all cases. The importance of the cis geometry of the leaving groups has been stressed in many studies that suggest the mechanism of antitumor activity of these compounds is related to their ability to inhibit replication by binding to adjacent guanine bases on duplex DNA. See, for example, Pinto et al, Biochem. Biophys. Acta. 1985 , 780 ,167 and Johnson et al, Biochemical Mechanisms of Platinum Antitumor Drugs, IRL Press, Washington, D. C., 1986, p.1.

Aside from those complexes conforming to the basic cis -$PtA_2X_2$ structural class, relatively few platinum compounds have demonstrated activity in in vivo tumor systems. U.S. Application Serial No. 723,783 filed April 16, 1985, and assigned to Engelhard, discloses a series of Pt antitumor agents which violate some of the classical structure-activity relationships. Triamine complexes of the formula I are disclosed:

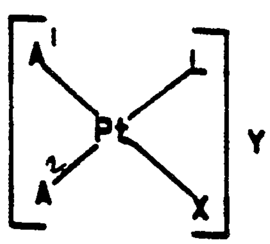

I

wherein A is a monodentate amine, such as $NH_3$ or $RNH_2$ (aliphatic amine), or a bidentate ($A_2$) ligand, such as ethylenediamine, and L is a heterocyclic unsaturated amine such as pyridine. Two other Engelhard patent applications, application Serial No. 077,269 filed July 24, 1987 and Serial No. 084,523 filed August 10, 1987, disclose platinum triamines where L is a saturated amine and where L is a nucleoside, respectively. There are no reports of platinum triamine compounds containing aniline or substituted anilines being tested for antitumor activity, However, the synthesis and characterization of several compounds of this type have been published. These include cis -$[Pt(NH_3)_2(L)Cl]Cl$ compounds in which L = aniline, 4-chloroaniline, 3-chloroaniline, 4-methylaniline, 4-methoxyaniline and 4-ethoxyaniline, cis -$[Pt(NH_3)_2(L)Br]Br$ compounds in which L = 4-methoxyaniline, 4-methylaniline, 4-ethoxyaniline and 3-methylaniline, and [Pt(en)(L)Cl]Cl in which L = aniline. See for example, Nee, M.; Roberts, J.D. Biochemistry 1982 , 21 , 4920.;

Petrosyan, V.S.; Popov. L.V.; Sakharov, S.G.; Zheligovskaya, N.N. Vestn. Mosk. Univ., Khim. 1975 , 16 , 91.; Ablov, A.V.; Roskina, N.N.; Chapurina, L.F.; Semina, V.G. Zh. Neorg. Khim. 1965 , 10 , 2230.; Ablov, A.V.; Semina, V.G. Zh. Neorg. Khim. 1965 , 10 , 608.; and Ablov, A.V.; Semina, V.G. Zh. Neorg. Khim. 1962 , 7 , 1801.

The above-mentioned U.S. patent applications correspond to European Applications No. 86302787.6 (EP-A-199,524), No. 88306758.9 (EP-A-300,812) and No. 88307341.3 (EP-A-303,437).

## Summary of the Invention

The present invention is directed to novel and active platinum(II) antitumor agents of the general formula(II):

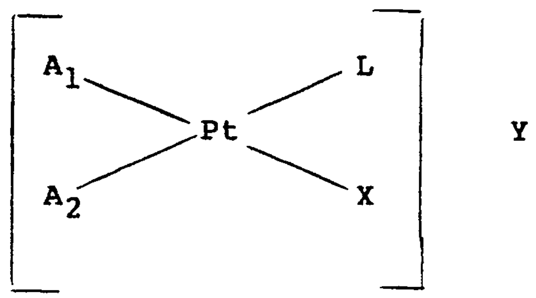
(II)

wherein the A groups, X and Y are as described in the prior art by the complex of formula I herein. L, however, is different from the L group of the prior art and is aniline or a substituted aniline. Members from this class of compounds have demonstrated activity in a number of in vivo antitumor screens, including the S180a and L1210 systems. These platinum-triamine complexes, which are typically cationic species, also possess desirable physical properties, such as high stability and solubility in aqueous media.

## Detailed Description of the Preferred Embodiment

The compounds of this invention are described by the following formula(II):

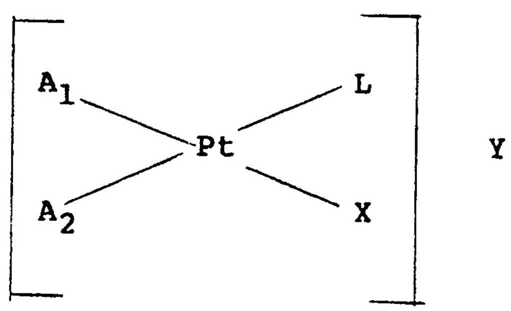
(II)

wherein $A^1$ and $A^2$ are the same or different and are selected from a monodentate amine, such as $NH_3$ or $R^1NH_2$, wherein $R^1$ is a linear or branched $C_1$-$C_6$ alkyl group which may bear one or more substituents selected from hydroxy, $C_1$-$C_4$ alkoxy, halo, carboxy, $C_1$-$C_4$ alkylcarboxy or $C_1$-$C_4$ alkoxycarbonyl. $A^1$ and $A^2$, taken together, represent a diamine of the general formula:

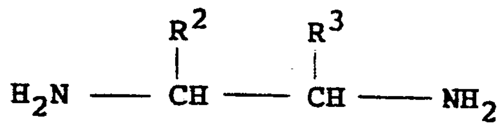

wherein $R^2$ and $R^3$, may be the same or different and are selected from a linear or branched $C_1$-$C_6$ alkyl group which may bear one or more substituents selected from hydroxy, $C_1$-$C_4$ alkoxy, halo, carboxy, $C_1$-$C_4$ alkylcarboxy or $C_1$-$C_4$ alkoxycarbonyl. $R^2$ and $R^3$ taken together may form a 1,2-diaminocycloalkane containing 4-8 nuclear carbon atoms which may be substituted on the carbons by one or more of the substituents selected from a linear or branched $C_1$-$C_6$ alkyl group, hydroxy, $C_1$-$C_4$ alkoxy, halo, carboxy, $C_1$-$C_4$ alkylcarboxy or $C_1$-$C_4$ alkoxycarbonyl.

L is aniline or aniline bearing one or more substituents selected from $C_1$-$C_6$ linear or branched alkyl, hydroxy, $C_1$-$C_4$ alkoxy, halo, carboxy, cyano, isocyano, phenyl, nitro, $C_1$-$C_4$ alkylcarboxy or $C_1$-$C_4$ alkoxycarbonyl.

X is a monodentate anionic ligand such as halo, hydroxo, pseudohalo, $HSO_4$, $H_2PO_4$, or $C_1$-$C_6$ carboxylate.

Y is an anion chosen from X above or nitrate or perchlorate. X may or may not be the same as Y.

## Pharmacology

The products of this invention either alone or in admixture with each other are useful in the treatment of malignant tumor cells sensitive thereto in animals as, for example, Sarcoma 180 ascites and L1210 leukemia in mammals such as mice. This antitumor effect also may extend to other sarcomas and leukemias and to such other tumor cells as lymphoid leukemia, lymphosarcoma, myelocytic leukemia, malignant lymphoma, squamous cell carcinoma, adenocarcinoma, scirrhous carcinoma, malignant melanoma, seminoma, teratoma, choriocarcinoma, embryonal carcinoma, cystadenocarcinoma, endometroidcarcinoma or neuroblastoma and the like. In addition, the complexes may be useful as anti-viral, anti-inflammatory, anti-bacterial and anti-parasitic agents.

The complexes of this invention may be administered parenterally or orally alone or in a mixture with a non-toxic pharmacologically acceptable inert carrier or diluent in any of the usual pharmaceutical forms. These include solid and liquid oral unit dosage forms such as tablets, capsules, powders and suspensions or solutions and suspensions for subcutaneous, intramuscular, intravenous, or intraarterial injection.

The term "unit dosage" refers to physically discrete units which may be administered in single or multiple dosages each containing a predetermined quantity of active ingredient in association with the required diluent, carrier or vehicle. The quantity of active ingredient is the amount of the complex which is needed to produce the desired therapeutic effect.

A typical unit dosage consists essentially of from about to 10-450 mg of active ingredient; however, the form in which the ingredient is administered and the frequency of administration is usually determinative of the concentration. Thus, for example, oral unit dosage forms containing 20 to 450 mg of active ingredient may be administered one or more times per day depending upon the severity of the tumor cells which is sought to be treated and the condition of the host animal. By contrast, parenteral administration generally requires from about 10 to about 100 mg of the active ingredient per unit dosage administered as a daily dose or as a fraction thereof depending upon whether the regimen calls for administration once, twice, three or four times daily.

By contrast to the unit dosage, the effective dose is that dosage which is needed to achieve the desired anti-tumor effect. In general, this dosage lies within the range of from about 10 to 950 mg of the active ingredient per kg of body weight of the host animal. A preferred concentration lies within the range of from about 30 to 450 mg/kg of body weight. For oral administration it has been found that an effective dose of about 50 to 950 mg/kg is most suitable, whereas in the case of parenteral administration it is usually advisable to employ from about 30 to 350 mg/kg. These unit dosages are well below the toxic or lethal dose and they may be varied over a wide range for adjustment to the patient who is being treated.

According to the present invention, the term "pharmacologically acceptable inert carrier or diluent" means a non-toxic substance which, when mixed with the active ingredient, renders it more suitable for administration. Compositions intended for oral administration may include such carriers or diluents as corn starch, potato starch, sodium carboxymethyl cellulose, ethyl cellulose, cellulose acetate, powder gum

tragacanth, gelatin, alginic acid, agar, stearic acid or the sodium, calcium and magnesium salts of stearic acid, sodium lauryl sulfate, polyvinylpyrrolidone, sodium nitrate, calcium carbonate and dicalciumphosphate. The compositions may also contain non-toxic adjuvants and modifiers such as dyes, buffering agents, preservatives, surfactants, emulsifiers, flavoring agents, biocides and the like.

Tablets are prepared by mixing a complex of this invention in a suitably comminuted or powdered form with a diluent or base such as starch, kaolin, dicalciumphosphate and the like. The resulting mixture can be granulated by wetting with a binder such as a syrup, starch (paste), acacia mucilage or solutions of cellulosic or polymeric materials, whereafter, the wetted mixture is formed through a screen. As an alternative to granulating, the powder mixture can be run through a tablet machine and any imperfectly formed slugs broken into granules. The granules are lubricated to prevent sticking to the tablet-forming dyes via the addition of stearic acid, a stearate salt, talc or mineral oil and the lubricated mixture is then compressed into tablets. The complexes can also be combined with free flowing inert carriers followed by compression into tablets without going through the granulated or slugging steps. A protective coating or sealing coat of shellac, sugar or polymeric material and a polished coating of wax can also be provided. Dye stuffs may be added to distinguish different unit dosages.

Dry or hard filled capsules are formulated by preparing a powdered mixture, according to the procedure herein before described and pouring the mixture into preformed gelatin sheets. A lubricant such as talc, magnesium stearate or calcium stearate can be added prior to the filling operation. A glidant such as coloidal silica may be added to improve the flow characteristics and a disintegrating or solubilizing agent may also be added to enhance the effectiveness of the medicament upon ingestion.

In soft gelatin capsules, the active complex is dissolved or suspended in vegetable oil, peanut oil, alcohol or glycerine and the like.

Powders for addition to foods, drinking water, fruit juice or other potable liquids are prepared by comminuting the compound to a fine size and mixing with a similarly comminuted pharmaceutical diluent or carrier such as an edible carbohydrate as, for example, starch. Sweetening agents and flavorings, preservatives and dispersing and/or coloring agents may also be employed.

Oral fluids such as syrups and elixirs are prepared in unit dosage forms so that a given quantity of medicament, such as a teaspoonful, will contain a predetermined amount of the active ingredient. Suspensions can be formulated by dispersing the active ingredient in a non-toxic vehicle in which it is essentially insoluble.

Compositions intended for parenteral administration may include such diluents and carriers as water or water-miscible solvents as, for example, sesame oil, groundnut oil, aqueous propylene glycol and a solution of sodium riboflavin. Typical of said compositions are solutions which contain the active ingredient in sterile form. Alternatively, a unit dosage form for parenteral administration can be prepared by placing a measured amount of complex in solution in sterile form, removing the solvent by lyophilization and sealing the vial. An accompanying vial of sterile vehicle can be provided for mixing with the complex prior to administration.

According to the present invention one or more of the complexes may be combined into a single unit dosage form or, alternatively, one or more of the complexes of the present invention may be combined with other known anti-tumor agents, therapeutic agents or nutrative agents so as to enhance or compliment the antitumor effect.

The preferred compositions for oral administration are tablets in which the complexes of the present invention are present in quantities of about 5 to about 375 mg but, preferably, about 10 to 200 mg in a pharmaceutically acceptable orally ingestible solid carrier. If desired, the compositions may also contain flavorants, binders, lubricants and other excipients known in the art.

A preferred alternative for oral administration is the soft gelatin capsule. Such a composition may contain from about 5 to about 375 mg, but, preferably, about 10 to 200 mg by weight of active ingredient dissolved or suspended in vegetable oral, peanut oil, alcohol or glycerin and the like.

A preferred form for parenteral administration will contain complexes of the present invention of about 10 to 100 mg, but preferably 15 to 75 mg.

The compounds of this invention may be prepared by first reacting: cis -$PtA_2Cl_2$ with an equimolar amount of $AgNO_3$ in DMF for 6-72 hr (24-48 hr preferred) at 0-50°C (20-30°C preferred.) The AgCl precipitate is removed by filtration and an equimolar amount of L is added to the filtrate. The mixture is allowed to react for 8-48 hr (16-24 hr preferred) at 0-50°C (20-30°C preferred). The solvent is then removed under vacuum. The materials are purified by shaking the residue with $CH_2Cl_2$ to remove excess L and DMF. cis -$PtA_2Cl_2$ and other impurities can be removed by recrystallizing the crude product from hot methanol followed by recrystallization from hot water.

## EXAMPLES

1. Compound Preparation .

The cis -[$Pt(NH_3)_2Cl_2$] starting material was prepared from $K_2[PtCl_4]$ (Engelhard) using the method described in Dhara Indian J. Chem. 1970 , 8 , 193, incorporated herein by reference. All other reagents were obtained from commercial sources.

### Example 1

**cis -[$Pt(NH_3)_2$(4-fluoraniline)Cl]$NO_3$**

Cisplatin (6.0g) and $AgNO_3$ (3.39g) were stirred in 100 mL DMF at room temperature while protected from light for 48 hr. Following removal of the AgCl precipitate by filtration, 4-fluoroaniline (1.9 mL) was added and the mixture was stirred at room temperature for 24 hr. The volume of the resulting solution was reduced to 5 mL under vacuum, 200 mL $CH_2Cl_2$ was added, and the mixture was shaken for 2 hr. The resulting solid was filtered and recrystallized three times form hot methanol and once from hot water. All recrystallizations were performed using decolorizing carbon. The resulting yield was 1.0g of a white crystalline solid. Anal. for $PtC_6H_{12}N_4O_3FCl$. Calcd: C, 16.46; H, 2.76; N, 12.80. Found: C, 16.45; H, 2.77; N, 12.73.

### Example 2

**cis -[$Pt(NH_3)_2$(4-chloroaniline)Cl]$NO_3$**

Cisplatin (6.0g) and $AgNO_3$ (3.39g) were stirred in 100 mL DMF for 48 hr at room temperature while protected from light. After removing the AgCl by filtration, 4-chloroaniline (2.55g) was added and the mixture stirred for an additional 24 hr. The volume of the solution was then reduced to 5-10 mL, 200 mL $CH_2Cl_2$ was added, and the mixture was shaken for 2 hr. The resulting solid was collected by filtration and recrystallized three times from hot methanol and once from hot water, employing decolorizing carbon for all recrystallizations. The yield was 0.73g (white solid). Anal for $PtC_6H_{12}N_4O_3Cl_{12}$. Calcd. C, 15.87; H, 2.66; N, 12.34. Found: C, 15.79; H, 2.81; N, 12.33.

### Example 3

**cis -[$Pt(NH_3)_2$(4-bromoaniline)Cl]$NO_3$**

Cisplatin (6.0g) and $AgNO_3$ (3.39g) were stirred in 100 mL DMF for 48 hr at room temperature while protected from light. After filtration of AgCl, 4-bromoaniline (3.44g) was added and stirring continued for 24 hr. The volume was then reduced to 5 mL under vacuum, 200 mL $CH_2Cl_2$ was added, and the mixture was shaken for 2hr. The resulting solid was collected by filtration and recrystallized three times from hot methanol and once from hot water, employing decolorizing charcoal in each case. The yield was 0.93g of a white solid. Anal. for $PtC_6H_{12}N_4O_3ClBr$. Calcd: C, 14.45; H, 2.43; N, 11.24. Found: C, 14.41; H, 2.46; N, 11.03.

### Example 4

**cis -[Pt($NH_3$)$_2$(4-iodoaniline)Cl]$NO_3$**

Cisplatin (6.0g) and $AgNO_3$ (3.39g) were stirred in 100 mL DMF for 24 hr. at room temperature with protection from light. The resulting AgCl was removed by filtration and 4-iodoaniline (4.38g) was added and stirring continued for 24 hr. Following this, the DMF was removed under vacuum, and the resulting oil treated by shaking with 150-200 mL $CH_2Cl_2$ for 2 hr. A solid was then filtered and recrystallized twice from hot methanol and once from water, employing decolorizing charcoal. The yield was 0.42g (light tan crystalline solid). Anal. for $PtC_6H_{12}N_4O_3ClI$. Calcd: C, 13.21; H, 2.22; N, 10.27. Found: C,13.24; H, 2.13; N, 10.22.

## Example 5

**cis -[Pt($NH_3$)$_2$(3-iodoaniline)Cl]$NO_3$**

Cisplatin (6.0g) and $AgNO_3$ (3.39g) were stirred in 100 mL DMF for 48 hr at room temperature while protected from light. After removing the AgCl by filtration, 3-iodoaniline (2.4 mL) was added and the stirring was continued for 24 hr. The volume of the solution was taken to 5-10 mL under vacuum, 150-200 mL $CH_2Cl_2$ was added, and the mixture was shaken for 2 hr. The resulting solid was collected by filtration and recrystallized twice from hot methanol and once from hot water, using decolorizing charcoal. The yield was 0.93g (white solid). Anal. for $PtC_6H_{12}N_4O_3ClI$. Calcd: C, 13.21; H, 2.22; N, 10.27. Found: C, 13.48; H, 2.31; N, 10.37.

The compounds were characterized by $^{195}Pt$ NMR (Table I), $^{13}C$ NMR (Table II) and by elemental analysis. HPLC was used to determine that the products were free of contamination by cis -$PtA_2Cl_2$, tetramine (cis-[$PtA_2L_2$]$X_2$) and free ligand L.

Table I

| $^{195}Pt$ NMR Data for Pt Triamines Containing Substituted Anilines | |
|---|---|
| Compound | Chemical Shift[a] |
| cis -[Pt($NH_3$)$_2$(4-fluoroaniline)Cl]$NO_3$ | -2372 |
| cis -[Pt($NH_3$)$_2$(4-chloroaniline)Cl]$NO_3$ | -2373 |
| cis -[Pt($NH_3$)$_2$(4-bromoaniline)Cl]$NO_3$ | -2369 |
| cis -[Pt($NH_3$)$_2$(4-iodoaniline)Cl]$NO_3$ | -2370 |
| cis -[Pt($NH_3$)$_2$(3-iodoaniline)Cl]$NO_3$ | -2365 |

[a]In $H_2O.D_2O$, vs $H_2PtCl_6$ 0 ppm.

Table II

| $^{13}C$ NMR Data for Pt Triamines Containing Substituted Anilines | |
|---|---|
| Compound | Chem. Shift[a] |
| cis -[Pt($NH_3$)$_2$(4-fluoroaniline)Cl]$NO_3$ | 136 †5 μ<br>116.3d<br>123.8d<br>159.5d |
| cis -[Pt($NH_3$)$_2$(4-chloroaniline)Cl]$NO_3$ | 131 †2<br>123.5<br>129.7<br>139.3 |
| cis -[Pt($NH_3$)$_2$(4-bromoaniline)Cl]$NO_3$ | 132 †7<br>123.9<br>132.6<br>119.0 |
| cis -[Pt($NH_3$)$_2$(4-iodoaniline)Cl]$NO_3$ | 140 †5<br>124.0<br>138.6<br>90.0 |
| cis -[Pt($NH_3$)$_2$(3-iodoaniline)Cl]$NO_3$ | 135 †5<br>131.2<br>130.8<br>121.5<br>93.8 |

[a]d = doublet; m = multiplet

## Antitumor Evaluation

All of the compounds of the Examples were tested against Sarcoma 180a in female CFW mice via single ip administration on Day 1. Two of the compounds were active in this system: cis -[Pt($NH_3$)$_2$(4-iodoaniline)Cl]NO3 (maximum ILS = 81% at 20 mg/kg) and cis -[Pt($NH_3$)$_2$(3-iodoaniline)Cl]$NO_3$ (maximum ILS = 83% at 20 mg/kg). The remaining compounds were not active. cis -[Pt($NH_3$)$_2$(4-iodoaniline)Cl]$NO_3$ was also tested against L1210 leukemia in female $CDF_1$ mice via single ip administration on Day 1. It was found to show a moderate level of activity (maximum ILS = 37% at 10 mg/kg). All of the results are shown in Tables III and IV.

While all of the compounds may not show activity in the screens used herein, there is no reason to believe that that would not test positive in other screens not exemplified herein.

## L1210 Screening Methodology

L1210 screening was conducted according to a published method. See Rose, et al, Cancer Treat. Repts ., 1982 , 66 , 135, which is incorporated herein by reference. Female $CDF_1$ mice (16-22g) were implanted with 1 x $10^6$ tumor cells ip on day zero. Compounds were administered in 0.5 mL of water ip on day one.

Groups of 6 mice were used for each dose. A control group of mice received only tumor and 0.5 mL of water. A positive control group received tumor and 8 mg/kg cisplatin in 0.5 mL of 0.15 M NaCl. The test was terminated after three times the mean survival time (MST) of the control group. Surviving mice were counted as dying on that day. Activity was determined based on the percent increase in MST of test mice over controls (%ILS). An ILS of > 25% represents activity. The tumor line was maintained through weekly transfer of 5 x $10^4$ cells in DBA/2 mice.

## Sarcoma 180 Ascites Screening Methodology

Female CFW mice (18-25g) were implanted with 2 x $10^6$ cells ip on day zero. Compounds were administered in 0.5 mL of water ip on day one. Groups of 6 mice were used for each test dose as well as controls as defined above. The test was run for twice the MST of the control group, with survivors counted as dying on that day. An ILS of > 50% indicates activity. The tumor line was maintained through weekly transfer of 4 x $10^6$ cells in CFW mice.

Table III

| S180a Antitumor Screening Data for Pt Triamines Containing Substituted Anilines | | | | | |
|---|---|---|---|---|---|
| Compound Vehicle | Dose mg/kg | %ILS | Surv | Positive %ILS | Control SURV |
| cis -[Pt($NH_3$)$_2$(4-fluoroaniline)Cl]$NO_3$ (water) | 1.25 | 6 | 0/6 | 98 | 6/6 |
| | 2.5 | 14 | 0/6 | | |
| | 5 | 27 | 0/6 | | |
| | 10 | 45 | 0/6 | | |
| | 20 | -48 | 1/6 | | |
| | 40 | -77 | 0/6 | | |
| cis -[Pt($NH_3$)$_2$(4-chloroaniline)Cl]$NO_3$ (water) | 1.25 | 3 | 0/6 | 98 | 6/6 |
| | 2.5 | 9 | 0/6 | | |
| | 5 | 40 | 0/6 | | |
| | 10 | 44 | 0/6 | | |
| | 20 | -51 | 0/6 | | |
| | 40 | -77 | 0/6 | | |
| cis -[Pt($NH_3$)$_2$(4-bromoaniline)Cl]$NO_3$ (water) | 1.25 | 2 | 0/6 | 92 | 4/6 |
| | 2.5 | 7 | 0/6 | | |
| | 5 | 38 | 0/6 | | |
| | 10 | 44 | 1/6 | | |
| | 20 | -14 | 2/6 | | |
| | 40 | -74 | 0/6 | | |
| cis -[Pt($NH_3$)$_2$(4-iodoaniline)Cl]$NO_3$ (water) | 0.63 | 12 | 0/6 | 95 | 5/6 |
| | 1.25 | -1 | 0/6 | | |
| | 2.5 | 22 | 0/6 | | |
| | 5 | 38 | 0/6 | | |
| | 10 | 39 | 0/6 | | |
| | 20 | 81 | 2/6 | | |
| cis -[Pt($NH_3$)$_2$(3-iodoaniline)Cl]$NO_3$ (water) | 1.25 | -3 | 0/6 | 92 | 4/6 |
| | 2.5 | 9 | 0/6 | | |
| | 5 | 23 | 0/6 | | |
| | 10 | 51 | 1/6 | | |
| | 20 | 83 | 2/6 | | |
| | 40 | 66 | 4/6 | | |

Table IV

| L1210 Antitumor Screening Data for Pt Triamine Containing Substituted Anilines | | | | | |
|---|---|---|---|---|---|
| Compound Vehicle | Dose mg/kg | %ILS | Surv | Positive %ILS | Control SURV |
| cis -[Pt($NH_3$)$_2$(4-iodoaniline)Cl]$NO_3$ | 1.25 | 14 | 0/6 | 190 | 5/6 |

## Claims

1: cis -[Pt($NH_3$)$_2$(4-fluroaniline)Cl]$NO_3$.
2: cis -[Pt($NH_3$)$_2$(4-bromoaniline)Cl]$NO_3$.
3: cis -[Pt($NH_3$)$_2$(4-iodoaniline)Cl]$NO_3$.
4: cis -[Pt($NH_3$)$_2$(3-iodoaniline)Cl]$NO_3$.
5: A composition for treating malignant animal tumors sensitive thereto comprising an active ingredient in a mixture with a non-toxic pharmacologically acceptable inert carrier or diluent, wherein the active ingredient is a compound of the general formula

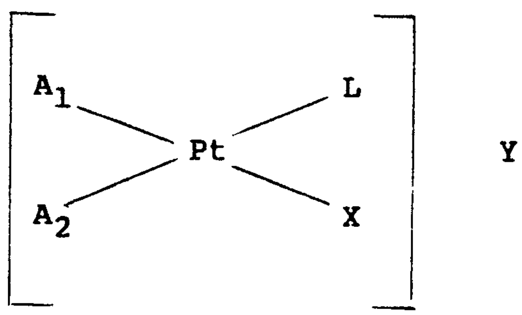
(II)

wherein $A_1$ and $A_2$ are the same or different and are selected from ammonia or a monodentate amine and substituted monodentate amines, or $A_1$ and $A_2$ together are a diamine represented by the general formula:

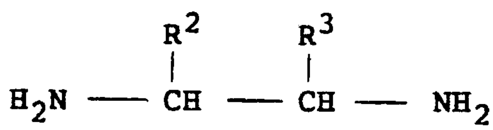

wherein $R_2$ and $R_3$ are the same or different and are each selected from hydrogen, linear or branched $C_1$-$C_6$ alkyl and substituted linear or branched $C_1$-$C_6$ alkyl, or $R_2$ and $R_3$, taken together, form a 1,2-diaminocycloalkane containing 4-8 nuclear carbon atoms or a substituted 1,2-diaminocycloalkane wherein the substituents are selected from linear or branched $C_1$-$C_6$ alkyl groups, hydroxy, $C_1$-$C_4$ alkoxy, halo, carboxy, $C_1$-$C_4$ alkylcarboxy or $C_1$-$C_4$ alkoxycarbonyl; wherein L is aniline or aniline substituted with one or more of $C_1$-$C_6$ linear or branched alkyl, hydroxy, $C_1$-$C_4$ alkoxy, halo, carboxy, cyano, isocyano, phenyl, nitro, $C_1$-$C_4$ alkylcarboxy or $C_1$-$C_4$ alkoxycarbonyl; wherein X is a monodentate anionic ligand; and wherein Y is an anion.
6: The composition according to Claim 5, wherein X is a monodentate anionic ligand selected from the group consisting of halo, hydroxo, pseudohalo, $HSO_4$, $H_2PO_4$, and $C_1$-$C_6$ carboxylate; and wherein Y is selected from the group consisting of halo, hydroxo, pseudohalo, $HSO_4$, $H_2PO_4$, $C_1$-$C_6$ carboxylate, nitrate and perchlorate.
7: A composition according to claim 5 or 6, wherein $A_1$ and $A_2$ are independently selected from $NH_3$ and $R_1NH_2$ groups, wherein $R_1$ is a substituted or unsubstituted, linear or branched $C_1$-$C_6$ alkyl group.
8: A composition according to claim 7, wherein the substituted linear or branched $C_1$-$C_6$ alkyl group $R_1$ contains one or more substituents selected from the group consisting of hydroxy, $C_1$-$C_4$ alkoxy, halo, carboxy, $C_1$-$C_4$ alkylcarboxy and $C_1$-$C_4$ alkoxycarbonyl.
9: A composition according to claim 5, which comprises an active ingredient selected from cis -[Pt($NH_3$)$_2$(4-fluoroaniline)Cl]$NO_3$, cis -[Pt($NH_3$)$_2$(4-bromoaniline)Cl]$NO_3$, cis -[Pt($NH_3$)$_2$(4-iodoaniline)Cl]$NO_3$ and cis -[Pt-($NH_3$)$_2$(3-iodoaniline)Cl]$NO_3$.
10: The composition according to claim 5, 6, 7 or 8, wherein $R_2$ and $R_3$ are the same or different and each is selected from hydrogen, a linear or branched $C_1$-$C_6$ alkyl group or a substituted linear or branched $C_1$-$C_6$ alkyl group wherein the substituents are selected from hydroxy, $C_1$-$C_4$ alkoxy, halo, carboxy, $C_1$-$C_4$ alkylcarboxy or $C_1$-$C_4$ alkoxylcarbonyl;
11: The composition of claim 5, 6, 7, 8 or 10, wherein the substituted 1,2-diaminocycloalkane is substituted with one or more substituents selected from the group consisting of hydroxy, $C_1$-$C_4$ alkoxy, halo, carboxy, $C_1$-$C_4$ alkylcarboxy and $C_1$-$C_4$ alkoxycarbonyl.
12: The composition of claim 5, wherein X is selected from the group consisting of halo, hydroxo, pseudohalo, $HSO_4$, $H_2PO4$, $C_1$-$C_6$ carboxylate, nitrate and perchlorate.
13: A method of making a composition according to any of claims 5 to 12, comprising mixing the said

active ingredient with the said diluent or carrier.

14: A method of treating malignant animal tumors sensitive to a compound of the general formula II as defined in any of claims 5 to 12, comprising administering the compound or a mixture of such compounds to an animal afflicted with such a tumor in an amount sufficient to cause regression of the tumor.

15: The method of claim 14, wherein the compound or mixture of compounds is administered orally.

16: The method of claim 14, wherein the compound or mixture of compounds is administered parenterally.

17. A compound of the general formula II as defined in any of claims 5 to 12, for use in the treatment of the human or animal body by therapy.

18. The use of a compound of the general formula II as defined in any of claims 5 to 12 in the manufacture of a medicament for treating animal malignant tumor cells.